# EUROPÄISCHE PATENTSCHRIFT

(11) **EP 2 001 837 B1**
(45) Veröffentlichungstag und Bekanntmachung des Hinweises auf die Patenterteilung: **25.12.2013**
(21) Anmeldenummer: 07724062.0
(22) Anmeldetag: 05.04.2007
(51) Int. Cl.: C07C 277/00, C07C 279/14, C07C 227/02

(54) **VERFAHREN ZUR HERSTELLUNG VON KREATIN, KREATIN-MONOHYDRAT ODER GUANIDINOESSIGSÄURE**
PROCESS FOR PREPARING CREATINE, CREATINE MONOHYDRATE OR GUANIDINOACETIC ACID
PROCEDE DE FABRICATION DE CREATINE, DE MONOHYDRATE DE CREATINE OU D'ACIDE GUANIDINOACETIQUE

(30) Priorität: 06.04.2006 DE 102006016227
(43) Veröffentlichungstag der Anmeldung: 17.12.2008
(73) Patentinhaber: AlzChem AG, 83308 Trostberg (DE)
(72) Erfinder: THALHAMMER, Franz, 83308 Trostberg (DE); GASTNER, Thomas, 84549 Engelsberg (DE)
(74) Vertreter: Dey, Michael
(86) Internationale Anmeldenummer: PCT/EP2007/003121
(87) Internationale Veröffentlichungsnummer: WO 2007/115799

(56) Entgegenhaltungen:
- EP-A- 1 382 390
- EP-A1- 0 513 396
- US-A- 5 739 390
- US-B1- 6 326 513

## Beschreibung

Gegenstand der vorliegenden Erfindung ist ein Verfahren zur Herstellung von Kreatin, Kreatin-Monohydrat oder Guanidinoessigsäure.

Guanidinoessigsäure und Kreatin sind bei Tieren und auch im Menschen vorkommende körpereigene Substanzen, wobei die Guanidinoessigsäure der direkte metabolische Vorläufer des Kreatins ist. Kreatin spielt im Energiestoffwechsel der Zelle eine wichtige Rolle. Im Körper entsteht durch Phosphorylierung von Kreatin das Phosphokreatin, welches neben dem Adenosintriphosphat (ATP) eine wesentliche Energiereserve des Muskels darstellt. Kreatin und Guanidinoessigsäure können sowohl endogen gebildet als auch durch die Nahrung aufgenommen werden, weshalb Kreatin seit langem als geeignetes Nahungsergänzungs- und Futtermittel bekannt ist. Auch Guanidinoessigsäure wurde bereits vorteilhaft als Futtermittel für Zucht und Masttiere zur Steigerung der Mastleistung und Verbesserung der Futterverwertung eingesetzt (WO 2005/120246 A1). Bei starker und über längere Zeit anhaltender Muskelarbeit sind die natürlich im Körper vorhandenen Kreatinvorräte rasch erschöpft. Aus diesem Grund haben sich insbesondere bei Leistungssportlern gezielte Kreatingaben positiv auf die Ausdauer und Leistungsfähigkeit ausgewirkt.

Synthesen zur Herstellung von Kreatin und Guanidinoessigsäure wurden bereits im 19. Jahrhundert entwickelt. Von Strecker wurde die Synthese von Guanidinoessigsäure aus Glycin und Cyanamid erstmals beschrieben (Strecker, M. Jahresber. Fortschr. Chem. Verw., (1861), 530). In Analogie konnte er nachfolgend auch Kreatin durch die Umsetzung von Sarkosin mit Cyanamid in wässriger Lösung erhalten (Strecker, Jahresber. über die Fortschritte der Chemie, (1868), 686).

In EP 0 754 679 B1 ist ein Verfahren zur Herstellung von Kreatin durch Umsetzung von Cyanamid mit Natrium- oder Kaliumsarkosinat beschrieben. Die Reaktion wird dabei in Wasser oder in einem Gemisch aus Wasser und einem organischen Lösemittel bei einer Temperatur von 20 bis 150 °C und einem pH-Wert von 7,0 bis 14,0 durchgeführt. Zur pH-Wert Einstellung wird eine organische oder anorganische Säure, vorzugsweise Salzsäure, Essigsäure oder Ameisensäure verwendet.

US 6,326,513 beschreibt die Umsetzung von Sarkosin oder Natriumsarkosinat mit S-Methylthioharnstoff oder mit S-Methylthioharnstoffsulfat in Wasser oder einer Mischung aus Wasser und einem Alkohol. Als Reaktionstemperatur sind 15 bis 140 °C vorgesehen; der pH-Wert beträgt 7,0 bis 13,0.

DE 199 20 962 A1 hat die Einstellung des pH-Wertes einer Sarkosin enthaltenden Lösung durch bipolare Elektodialyse zum Gegenstand. Die hergestellte Lösung wird hierbei vorzugsweise auf einen pH-Wert von 9 bis 10 eingestellt und kann im Folgenden mit Cyanamid zu Kreatin oder Kreatin Monohydrat umgesetzt werden.

Weiterhin beschreibt das Patent CN 1240207 die Herstellung einer Sarkosinat-Lösung aus Chloressigsäure und Methylamin. Zur Herstellung von Kreatin wird das erhaltene Sarkosinhydrochlorid mit Natronlauge und Ammoniak auf einen pH-Wert von 9-12 eingestellt und mit Cyanamid umgesetzt.

Die Herstellung von Natriumsarkosinat über eine Strecker-Synthese ist seit langem bekannt. So beschreibt DE 25 03 582 A1 die Umsetzung von Methylamin, Formaldehyd und Blausäure bei 18°C und die anschließende Verseifung des gebildeten Sarkosinnitrils mit Natronlauge oder Kalilauge bei 50-55°C.

Auch die Herstellung wässriger Lösungen von Natriumsalzen der Aminosäuren Glycin und Sarkosin durch katalytische Dehydrierungsreaktion der entsprechenden Aminoalkohole Ethanolamin bzw. N-Methylethanolamin ist bekannt. Nach dem Stand der Technik wird die Dehydrierung von Aminoalkoholen in wässriger, alkalischer Lösung an einem kupferhaltigen Katalysator bei erhöhter Temperatur und unter Druck durchgeführt. Der während der Reaktion gebildete Wasserstoff wird dabei aus dem Reaktor abgelassen, um den Druck konstant zu halten. Für die Reaktion werden unterschiedliche Katalysator-Typen eingesetzt, wobei allen gemeinsam ist, dass sie als aktives Element Kupfer enthalten. So beschreibt die US-Anmeldung US 2002-038,051 die Verwendung von Raney-Kupfer, das vorzugsweise mit anderen Metallen, insbesondere Edelmetallen dotiert ist. Nach diesem Verfahren wird Na-Glycinat mit einer Ausbeute von > 98 % erhalten. Auch von der Herstellung von Na-Sarkosinat wird berichtet.

Das Patent US 6159894 beansprucht die Verwendung eines Katalysators auf Basis von Kupfer und Zirkonium, der gegebenenfalls mit anderen Metallen dotiert ist, um Aminocarbonsäuren aus Aminoalkoholen herzustellen.
Die Anmeldeschrift WO 98/50150 A1 befasst sich mit der Dehydrierung von u.a. Ethanolaminen an einem Kupfer-basierten Katalysator auf einem inerten Träger, wie beispielsweise Aktivkohle.

Die Verwendung einer nach dem Stand der Technik erhaltenen Reaktionslösung von Na-Sarkosinat oder Na-Glycinat aus einem Dehydrierungsprozess zur Herstellung von Kreatin bzw. Guanidinoessigsäure ist bislang nicht bekannt.

Die nach dem Stand der Technik verwendeten Natriumsarkosinat- oder Sarkosin-Lösungen bzw. Natriumglycinat- und Glycin-Lösungen für die Herstellung von Kreatin, Kreatin Monohydrat und Guanidinoessigsäure werden nach dem Strecker-Verfahren oder aus Chloressigsäure und Methylamin bzw. Ammoniak hergestellt und enthalten in der Regel charakteristische Verunreinigungen wie Blausäure, Formaldehyd, Chloressigsäure, Iminodiessigsäure, Methyliminodiessigsäure, Ammoniak und Methylamin. Dieses Verunreinigungsspektrum führt im Herstellprozess von Kreatin, Kreatin Monohydrat und Guanidinoessigsäure aber zu Problemen, da diese Verbindungen oder Folgeprodukte wie etwa Dihydrotriazin als Verunreinigungen im Endprodukt verbleiben können und aus toxikologischer Sicht bedenklich sind.

Der vorliegenden Erfindung lag daher die Aufgabe zugrunde, ein neues Verfahren zu entwickeln, welches die genannten Nachteile entsprechend dem Stand der Technik nicht aufweist, sondern die Herstellung von Kreatin, Kreatin-Monohydrat oder Guanidinoessigsäure in höchsten Ausbeuten und Reinheiten ermöglicht.

Die Aufgabe wurde erfindungsgemäß dadurch gelöst, dass a₁) N-Methylethanolamin in alkalischer Lösung an einem Katalysator zu Natriumsarkosinat oder a₂) Ethanolamin in alkalischer Lösung an einem Katalysator zu Natriumglycinat dehydriert werden, anschließend b) die so aus a₁) erhaltene Natriumsarkosinat-Lösung bzw. die aus a₂) erhaltene Natriumglycinat-Lösung durch eine Säure oder durch bipolare Elektrodialyse auf einen pH-Wert von 7,0 bis 13,0 eingestellt werden und das so erhaltene Sarkosin bzw. Glycin mit Cyanamid als Guanylierungsmittel umgesetzt werden, wobei die einzelnen Stufen ohne die Isolierung von Zwischenprodukten durchgeführt werden. Überraschend hat sich gezeigt, dass sich mit Natriumsarkosinat bzw. Natriumglycinat, welche durch katalytische Dehydrierung von Methylethanolamin bzw. Ethanolamin hergestellt wurden, die geschilderten Probleme nach dem Stand der Technik umgehen lassen. Die Bildung von Blausäure, Formaldehyd, Chloressigsäure und Ammoniak oder Methylamin wird vollständig vermieden. Überraschend hat sich auch gezeigt, dass die Bildung von Dihydrotriazin bei der Kreatin-Herstellung nicht mehr zu beobachten ist, wenn katalytisch hergestelltes Natriumsarkosinat verwendet wird. Weiterhin wird die Belastung der Endprodukte mit Iminodiessigsäure oder Methyliminodiessigsäure deutlich reduziert. Diese Vorteile waren in ihrer Gesamtheit so nicht zu erwarten.

Die Erfindung betrifft insbesondere ein Verfahren zur Herstellung von Kreatin, Kreatin-Monohydrat oder Guanidinoessigsäure, wobei zunächst N-Methylethanolamin oder Ethanolamin in jeweils alkalischer Lösung katalytisch dehydriert werden und abschließend die so erhaltenen Sarkosinat- bzw. Glycinat-Lösungen unter sauren Bedingungen mit Cyanamid als Guanylierungsmittel umgesetzt werden, wobei die einzelnen Stufen ohne die Isolierung von Zwischenprodukten durchgeführt werden. Auf diese Weise werden Produkte in hohen Ausbeuten
und sehr guter Reinheit erhalten, wobei im Gegensatz zum Stand der Technik keinerlei Spuren von Blausäure, Formaldehyd, Chloressigsäure oder Ammoniak enthalten sind. Auch wird die Bildung des toxikologisch bedenklichen Dihydrotriazins vermieden.
Für die katalytische Dehydrierung von Ethanolamin und Methylethanolamin gemäß Verfahrensstufe a) eignen sich die aus dem Stand der Technik bekannten kupferhaltigen Katalysatoren. Diese können auch mit weiteren Metallen dotiert sein, wie etwa Nickel, Palladium oder Platin. Aber auch reine Nickel-, Palladium- oder Platin-Katalysatoren sind hervorragend geeignet, wobei die verwendeten Katalysatoren jeweils auch in geträgerter Form eingesetzt werden können. Die Reaktion wird bei Temperaturen zwischen 120 und 220 °C durchgeführt, wobei Drücke zwischen 0,01 und 30 bar, vorzugsweise zwischen 0,1 und 20 bar und besonders bevorzugt zwischen 1,0 und 10 bar empfehlenswert sind. Vorteilhaft wird hierbei in wässrigen Lösungen gearbeitet, wobei die Konzentrationen an Methylethanolamin oder Ethanolamin aus wirtschaftlichen Gründen in der Regel so gewählt werden, dass nach der Reaktion gemäß den Stufen a₁) bzw a₂) eine 20 bis 60%ige Lösung des Produktes vorliegt. Lösungen mit einer Konzentration zwischen 30 und 45 % sind als besonders bevorzugt anzusehen. Alle Prozentangaben hierin beziehen sich auf das Gewicht, soweit nichts anderes angegeben ist.

Es ist vorteilhaft, den Katalysator vor dem folgenden Reaktionsschritt b) abzutrennen, da die abschließende Umsetzung mit einem Guanylierungsmittel im beschriebenen Konzentrationsbereich zum Präzipitieren des gebildeten Produktes führt.

Für die nachfolgende pH-Einstellung der erhaltenen Natriumsarkosinat- oder Natriumglycinat-Lösungen auf Werte zwischen 7 und 13 eignen sich Salpetersäure, Phosphorsäure, Essigsäure, Salzsäure, Schwefelsäure und/oder Kohlendioxid. Alternativ kann der pH-Wert auch mit Hilfe einer bipolaren Elektrodialyse eingestellt werden. Die hierbei entstehende Base kann nämlich in die erste Verfahrensstufe zurückgeführt werden, was von der vorliegenden Erfindung ebenfalls berücksichtigt wird.

Zur Guanylierung der aus Stufe a₁) erhaltenen Sarkosin- bzw. der aus der Stufe a₂) erhaltenen Glycin-Lösung wird Cyanamid als Guanylierungsmittel verwendet.
Die Guanylierung kann gemäß vorliegender Erfindung in einem Temperaturbereich von 10° bis 120 °C, vorzugsweise zwischen 20 und 80 °C, und bei Drücken von 0,1 bis 10 bar, vorzugsweise von 1,0 bis 5,0 bar, durchgeführt werden. Es ist im Rahmen der Erfindung als bevorzugt anzusehen, das Verhältnis von Natriumsarkosinat bzw. Sarkosin oder Natriumglycinat bzw. Glycin zum Guanylierungsmittel zwischen 1 : 0,1 bis 2,0 zu wählen. Bevorzugt beträgt das Verhältnis 1 : 1 bis 1,1..

Das beschriebene Verfahren wird ohne die Isolierung der gebildeten Zwischenprodukte durchgeführt; auch eignet es sich für eine kontinuierliche Prozessführung.

Das gebildete Produkt in Form von Kreatin oder Guanidinoessigsäure fällt als Suspension an und kann mit jeder geläufigen Art und Weise von der flüssigen Phase abgetrennt werden. Die erhaltenen Kristalle werden im Anschluss vorzugsweise mit einem wässrigen Medium gewaschen und getrocknet. Je nach Vorgehen bei der Trocknung wird Kreatin oder Kreatin Monohydrat erhalten. Die Guanidinoessigsäure bildet hingegen kein stabiles Monohydrat.

Es hat sich insgesamt gezeigt, dass man mit dem erfindungsgemäßen Verfahren das gewünschte Reaktionsprodukt nicht nur gemäß Aufgabenstellung in hohen Ausbeuten und sehr guter Reinheit erhält, sondern dass insbesondere die Raum/Zeit-Ausbeuten äußerst gut sind, was das Verfahren gleichzeitig mit einer vereinfachten Prozessführung sehr wirtschaftlich macht.
Die nachfolgenden Beispiele verdeutlichen die Breite der vorliegenden Erfindung.

### Beispiele

### Beispiel 1: Herstellung einer Na-Sarkosinat-Lösung

In einem 600 ml Autoklav mit Rührer, Druckregelventil und Mantelbeheizung wurden 10 g (berechnet auf Trockensubstanz) eines Raney-Katalysators auf Basis Cu/Ni (Degussa AG, BOO 111) als Suspension in Wasser vorgelegt und 75 g N-Methylethanolamin, sowie 204 g einer 20 Gew.-%igen Natronlauge zugegeben. Der Autoklav wurde verschlossen und der Inhalt mit Hilfe der Mantelbeheizung auf eine Temperatur von 160 °C aufgeheizt. Dabei bildete sich ein zunehmender Druck von Wasserstoff, der durch geregeltes Öffnen des Druckhalteventils bei 10 bar (absolut) gehalten wurde. Nach 4 Stunden war die Gasfreisetzung vollständig beendet und der Reaktor wurde auf 90 °C abgekühlt. Nach Abstellen des Rührers sedimentierte der Katalysator innerhalb einer Stunde und ein Teil der überstehenden klaren Lösung von Na-Sarkosinat wurde über ein Tauchrohr aus dem Autoklaven abgenommen. Der Katalysator verblieb im Reaktor und konnte in weiteren Ansätzen erneut eingesetzt werden.

Für jeden weiteren Zyklus wurde die im Reaktor verbleibende Suspension des Katalysators mit den genannten Mengen an
N-Methylethanolamin und Natronlauge versetzt und durch Erwärmen die Reaktion gestartet.

Mit Ausnahme des Startansatzes erhielt man pro Zyklus 270 g einer farblosen Lösung von Na-Sarkosinat mit einem Gehalt von 40,0 Gew.-% (entspricht einer Ausbeute von 97,2 % d.Th.).

### Beispiel 2: Herstellung einer Lösung von Na-Glycinat

Analog Beispiel 1 wurden in einem Autoklav 10 g(berechnet auf Trockensubstanz) eines mit Pd dotierten Kupferkatalysators auf Aktivkohle (Degussa AG, CE 1015 OY/W) suspendiert in Wasser vorgelegt und 61 g Ethanolamin, sowie 272 g Natronlauge (15 Gew.-%ig) zugegeben. Der Autoklav wurde verschlossen und der Inhalt auf 160 °C aufgeheizt. Der Druck wurde bei 12 bar (absolut) gehalten und der entstehende Wasserstoff über ein Druckregelventil kontinuierlich abgelassen. Nach Reaktionsende (erkennbar am Abklingen der Gasfreisetzung) wurde auf 90 °C abgekühlt und der Katalysator von der Produktlösung abfiltriert. Man erhielt 315 g Filtrat, das 29;8 Gew.-% Na-Glycinat enthielt (entspricht einer Ausbeute von 96,7 % d.Th.).

Der abfiltrierte Katalysator wurde in 10 g Wasser suspendiert und in weitere Folgeansätze, zurückgeführt.

### Beispiel 3: Herstellung von Kreatin Monohydrat

4625 g (16,7 mol) einer nach Beispiel 1 hergestellten 40 Gew.-%igen Natriumsarkosinat-Lösung wurden vorgelegt. Unter äußerer Kühlung mit kaltem Wasser und kräftigem Rühren wurde mit konzentrierter Salzsäure ein pH-Wert von 9,6 (bei 20 °C) eingestellt. Anschließend wurde auf 75 °C erhitzt. 1403 g (16,7 mol) einer 50 gew.-%-igen wässrigen Cyanamid-Lösung (Degussa-Cyanamid L 500) wurden während 90 Minuten unter starkem Rühren gleichmäßig bei einer Innentemperatur von 75 bis 80 °C eingetragen. Nach Beendigung der Cyanamid-Zugabe wurde das Reaktionsgemisch noch 2 Stunden bei einer Innentemperatur von 75 °C gerührt. Nach dem Abkühlen auf 5 °C wurde das kristalline, gut filtrierbare Reaktionsprodukt abgesaugt, dreimal mit jeweils 1250 ml Wasser chloridfrei gewaschen und im Vakuumtrockenschrank bei 40 °C und 20 mbar getrocknet. Die Ausbeute betrug 1897 g Kreatin Monohydrat (entspricht einer Ausbeute von 76,2 % d.Th.).

## Patentansprüche

1. Verfahren zur Herstellung von Kreatin, Kreatin-Monohydrat oder Guanidinoessigsäure, **dadurch gekennzeichnet, dass**
a₁) N-Methylethanolamin in alkalischer Lösung an einem Katalysator zu Natriumsarkosinat
oder
a₂) Ethanolamin in alkalischer Lösung an einem Katalysator zu Natriumglycinat,
dehydriert werden,
anschließend
b) die so aus a₁) erhaltene Natriumsarkosinat-Lösung bzw. die aus a₂) erhaltene Natriumglycinat-Lösung durch eine Säure oder durch bipolare Elektrodialyse auf einen pH-Wert von 7,0 bis 13,0 eingestellt werden und das so erhaltene Sarkosin bzw. Glycin mit Cyanamid als Guanylierungsmittel umgesetzt werden,
wobei die einzelnen Stufen ohne die Isolierung von Zwischenprodukten durchgeführt werden.

2. Verfahren nach Anspruch 1,
**dadurch gekennzeichnet,**
**dass** die katalytische Dehydrierung bei einem Druck von 0,01 bis 30 bar, vorzugsweise 0,1 bis 20 bar und besonders bevorzugt 1,0 bis 10 bar und einer Temperatur von 120 bis 220°C durchgeführt wird.

3. Verfahren nach einem der Ansprüche 1 oder 2,
**dadurch gekennzeichnet,**
**dass** die Verfahrensstufe a) in Gegenwart mind. eines Katalysators der Reihe Kupfer, Nickel, Palladium oder Platin, vorzugsweise jeweils in geträgerter und besonders bevorzugt in dotierter Form durchgeführt wird.

4. Verfahren nach einem der Ansprüche 1 bis 3,
**dadurch gekennzeichnet,**
**dass** die aus Verfahrensstufe a₁) erhaltene Natriumsarkosinat-Lösung bzw. die aus Stufe a₂) erhaltene Natriumglycinat-Lösung eine Konzentration von 20 bis 60 Gew.-% und vorzugsweise von 30 bis 45 Gew.-% aufweist.

5. Verfahren nach einem der Ansprüche 1 bis 4,
**dadurch gekennzeichnet,**
**dass** die Katalysator-Komponente vor der Verfahrensstufe b) aus dem Reaktionssystem entfernt wird.

6. Verfahren nach einem der Ansprüche 1 bis 5,
**dadurch gekennzeichnet,**
**dass** in Verfahrensstufe b) als Säure Salpetersäure, Phosphorsäure, Essigsäure, Salzsäure, Schwefelsäure und/oder Kohlendioxid eingesetzt werden.

7. Verfahren nach einem der Ansprüche 1 bis 5,
**dadurch gekennzeichnet,**
**dass** die bei der bipolaren Elektrodialyse gewonnene Base zur pH-Wert-Einstellung in die Verfahrensstufe a) rückgeführt wird.

8. Verfahren nach einem der Ansprüche 1 bis 7,
**dadurch gekennzeichnet,**
**dass** die Guanylierung bei einem Druck von 0,1 bis 10 bar, vorzugsweise bis 1,0 bis 5,0 bar, und einer Temperatur von 10 bis 120 °C, vorzugsweise von 20 bis 80 °C durchgeführt wird.

9. Verfahren nach einem der Ansprüche 1 bis 8,
**dadurch gekennzeichnet,**
**dass** das Molverhältnis von Natriumsarkosinat bzw. Sarkosin oder Natriumglycinat bzw. Glycin zum Guanylierungsmittel 1 : 0,1 bis 2,0 und bevorzugt 1 : 1,0 bis 1,1 beträgt.

10. Verfahren nach einem der Ansprüche 1 bis 9,
**dadurch gekennzeichnet,**
**dass** es kontinuierlich durchgeführt wird.

11. Verfahren nach einem der Ansprüche 1 bis 10,
**dadurch gekennzeichnet,**
**dass** das erhaltene Produkt abschließend vorzugsweise mit einem wässrigen Medium gewaschen wird.

## Claims

1. Process for producing creatine, creatine monohydrate or guanidinoacetic acid, **characterized in that**
a₁) N-methylethanolamine is dehydrogenated in alkaline solution on a catalyst to form sodium sarcosinate
or
a₂) ethanolamine is dehydrogenated in alkaline solution on a catalyst to form sodium glycinate
after which
b) the sodium sarcosinate solution obtained in this manner from a₁) or the sodium glycinate solution obtained from a₂) is adjusted to a pH of 7.0 to 13.0 by an acid or by bipolar electrodialysis and the sarcosine or glycine obtained in this manner is reacted with cyanamide as a guanylating agent,
wherein the individual steps are carried out without isolating intermediate products.

2. Process according to claim 1,
**characterized in that**
the catalytic dehydrogenation is carried out at a pressure of 0.01 to 30 bar, preferably of 0.1 to 20 bar and particularly preferably of 1.0 to 10 bar and at a temperature of 120 to 220°C.

3. Process according to one of the claims 1 or 2,
**characterized in that**
the process step a) is carried out in the presence of at least one catalyst of the group comprising copper, nickel, palladium or platinum, each of which is preferably in a supported form and particularly preferably in a doped form.

4. Process according to one of the claims 1 to 3,
**characterized in that**
the sodium sarcosinate solution obtained from process step a₁) or the sodium glycinate solution obtained from step a₂) has a concentration of 20 to 60 % by weight and preferably of 30 to 45 % by weight.

5. Process according to one of the claims 1 to 4,
**characterized in that**
the catalyst component is removed from the reaction system before process step b).

6. Process according to one of the claims 1 to 5,
**characterized in that**
nitric acid, phosphoric acid, acetic acid, hydrochloric acid, sulphuric acid and/or carbon dioxide are used as an acid in process step b).

7. Process according to one of the claims 1 to 5,
**characterized in that**
the base obtained in the bipolar electrodialysis is fed back into process step a) to adjust the pH.

8. Process according to one of the claims 1 to 7,
**characterized in that**
the guanylation is carried out at a pressure of 0.1 to 10 bar, preferably up to 1.0 to 5.0 bar and at a temperature of 10 to 120°C, preferably of 20 to 80°C.

9. Process according to one of the claims 1 to 8,
**characterized in that**
the molar ratio of sodium sarcosinate or sarcosine or sodium glycinate or glycine to the guanylating agent is 1 : 0.1 to 2.0 and preferably 1 : 1.0 to 1.1.

10. Process according to one of the claims 1 to 9,
**characterized in that**
it is conducted as a continuous process.

11. Process according to one of the claims 1 to 10,
**characterized in that**
the obtained product is subsequently washed preferably with an aqueous medium.

## Revendications

1. Procédé de fabrication de créatine, de monohydrate de créatine ou d'acide guanidinoacétique, **caractérisé en ce que**
a₁) de la N-méthyléthanolamine en solution alcaline est déshydrogénée en sarcosinate de sodium sur un catalyseur
ou
a₂) de l'éthanolamine en solution alcaline est déshydrogénée en glycinate de sodium sur un catalyseur,
ensuite
b) la solution de sarcosinate de sodium ainsi obtenue en a₁), respectivement la solution de glycinate de sodium obtenue en a₂) est ajustée à une valeur de pH comprise entre 7,0 et 13,0 à l'aide d'un acide ou par électrodialyse bipolaire, et la sarcosine respectivement la glycine ainsi obtenue est mise en réaction avec du cyanamide comme agent de guanylation,
les différentes étapes étant réalisées sans l'isolation des produits intermédiaires.

2. Procédé selon la revendication 1
**caractérisé en ce que**
la déshydrogénation catalytique est réalisée sous une pression de 0,01 à 30 bar, de préférence de 0,1 à 20 bar et avec une préférence particulière de 1,0 à 10 bar, et à une température de 120 à 220 °C.

3. Procédé selon l'une des revendications 1 ou 2,
**caractérisé en ce que**
l'étape de procédé a) est réalisée en présence d'au moins un catalyseur de la série du cuivre, du nickel, du palladium ou du platine, de préférence dans chaque cas sous forme supportée et avec une préférence particulière, sous forme dopée.

4. Procédé selon l'une des revendications 1 à 3,
**caractérisé en ce que**
la solution de sarcosinate de sodium obtenue par l'étape de procédé a₁), respectivement la solution de glycinate de sodium obtenue par l'étape de procédé a₂), présente une concentration de 20 à 60 % en poids et de préférence, de 30 à 45 % en poids.

5. Procédé selon l'une des revendications 1 à 4,
**caractérisé en ce que**
le composant catalyseur est retiré du système réactionnel avant l'étape de procédé b).

6. Procédé selon l'une des revendications 1 à 5,
**caractérisé en ce que**
l'on utilise comme acide dans l'étape de procédé b), l'acide nitrique, l'acide phosphorique, l'acide acétique, l'acide chlorhydrique, l'acide sulfurique et/ou le dioxyde de carbone.

7. Procédé selon l'une des revendications 1 à 5,
**caractérisé en ce que**
la base obtenue lors de l'électrodialyse bipolaire est recyclée dans l'étape de procédé a) pour l'ajustement de la valeur du pH.

8. Procédé selon l'une des revendications 1 à 7,
**caractérisé en ce que**
la guanylation est réalisée sous une pression de 0,1 à 10 bar, de préférence de 1,0 à 5,0 bar, et à une température de 10 à 120 °C, de préférence de 20 à 80 °C.

9. Procédé selon l'une des revendications 1 à 8,
**caractérisé en ce que**
le rapport molaire entre le sarcosinate de sodium respectivement la sarcosine ou entre le glycinate de sodium respectivement la glycine et l'agent de guanylation s'élève à 1:0,1 à 2,0 et de préférence, à 1:1,0 à 1,1.

10. Procédé selon l'une des revendications 1 à 9,
**caractérisé en ce qu'**il
est réalisé en continu.

11. Procédé selon l'une des revendications 1 à 10,
**caractérisé en ce que**
le produit obtenu est soumis à un lavage terminal, de préférence avec un milieu aqueux.
